Europäisches Patentamt

European Patent Office

Office européen des brevets

(11) Publication number: **0 533 724 B1**

# EUROPEAN PATENT SPECIFICATION

(45) Date of publication of patent specification: **26.04.95**

(51) Int. Cl.[6]: **C07C 59/265**, A61K 31/19, C07C 59/245, C07C 51/41

(21) Application number: **91910588.2**

(22) Date of filing: **30.05.91**

(86) International application number: **PCT/US91/03779**

(87) International publication number: **WO 91/19692 (26.12.91 91/29)**

(54) CALCIUM CITRATE MALATE COMPOSITION.

(30) Priority: **14.06.90 US 537313**
**31.10.90 US 608055**

(43) Date of publication of application: **31.03.93 Bulletin 93/13**

(45) Publication of the grant of the patent: **26.04.95 Bulletin 95/17**

(84) Designated Contracting States: **AT BE DE FR GB IT NL**

(56) References cited:
**EP-A- 0 297 679**
**EP-A- 0 304 987**

(73) Proprietor: **THE PROCTER & GAMBLE COMPANY**
**One Procter & Gamble Plaza**
**Cincinnati**
**Ohio 45202 (US)**

(72) Inventor: **FOX, Mary, Mora**
**13 Applewood Dr.**
**Fairfield, OH 45014 (US)**
Inventor: **HECKERT, David, Clinton**
**3561 Kehr Road**
**Oxford, OH 45056 (US)**
Inventor: **LUHRSEN, Kenneth, Ray**
**Route 1, Box 11**
**Aurora, IN 47001 (US)**

(74) Representative: **Canonici, Jean-Jacques et al**
**Procter & Gamble European Technical Center N.V.**
**Temselaan 100**
**B-1853 Strombeek-Bever (BE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

**Description**

This invention relates to a soluble calcium source comprising specific molar ratios of calcium, citrate and malate. The composition is more soluble than calcium citrate or calcium malate alone, and when prepared by the process herein, consists of a metastable complex salt which is distinct from either pure calcium citrate or pure calcium malate.

BACKGROUND OF THE INVENTION

Calcium is a mineral that is important for building bones and teeth. One of the problems with supplementation of the diet with calcium is that all sources of calcium are not equally soluble or bioavailable. In addition, some calcium sources are not as pure as other sources. For example, calcium carbonate derived from bone meal, oyster shell, or other biological origins contains trace amounts of lead and other minerals. Some calcium carbonates also contain silica. Therefore, it is necessary to take additional amounts of these materials to achieve the same calcium level as those taken from synthetic sources which are essentially pure calcium carbonate.

Calcium citrate is poorly soluble in water; 1 gram of calcium citrate dissolves per 1050 grams of cold water. Calcium malate exhibits a similar solubility. Calcium hydroxide is only slightly soluble in water, and it absorbs carbon dioxide from the air readily forming calcium carbonate.

In addition, calcium salts readily hydrate even when stored in dry cool places. Therefore, the amount of calcium being delivered may be even less than expected because of the large amount of water absorbed by the salts.

Therefore a calcium salt which is readily bioavailable and which is readily soluble in neutral solutions as well as acidic solutions is highly desirable. This would allow the calcium salt to be added to neutral as well as acid foods in a soluble form and to be an effective supplement. Granular salts can produce a gritty taste in foods and beverages if they are not soluble or are too large in particle size.

It is therefore an object of this invention to produce a calcium salt which is highly bioavailable and which is readily soluble in water.

BACKGROUND ART

Japanese patent application Sho 56/097,248 (Tanaka, 1981) discloses a calcium citrate malate salt of increased solubility. This salt is a 5:2:2 ratio of calcium:citrate:malate. The applicant suggests that 2 molecules of calcium malate are associated with 1 molecule of calcium citrate. The product is described as a white crystalline powder which is soluble at 0.5 g/100 ml of water at 20°C and which has a pH of 6 to 6.5. The product is formed by mixing calcium carbonate with citric and malic acids in water at 50°C to 60°C and separating the white crystalline material from the mother liquor. When the product is dry (oven drying is used) the temperature is raised to 110°C-120°C to sterilize the powder. Alternatively a product is made using calcium chloride, sodium citrate and sodium malate.

Calcium citrate malate has been made in beverages. U.S. 4,737,375 issued to Nakel et al describes beverages nutritionally supplemented with calcium to which citric and malic acids are added.

U.S. 4,722,847 issued to Heckert (1988) describes calcium citrate malate in beverages and the preparation of the material in a beverage format.

European Patent to Jacobs entitled "Novel Calcium Supplements", 304,987 (1989), discloses calcium citrate malate materials that have a 6:2:3 molar ratio.

SUMMARY OF THE INVENTION

A process for forming a metastable calcium citrate malate having a solubility in water at about 20°C of 75% or more weight percent calcium per calcium citrate malate on a 100 mg basis comprising:
(a) adding a calcium source selected from the group consisting of calcium hydroxide, calcium carbonate, or calcium oxide to citric and malic acid in water, preferably wherein the mole ratios of calcium is from 2 to 10, of citric is from 1 to 3 and of malic acid is from 1 to 5;
(b) mixing this solution at 20°C to about 80°C until the citric and malic acids are neutralized by the calcium, preferably at a temperature of from 30°C to 80°C;
(c) drying the mixture at a temperature of less than 100°C; and
(d) grinding the resultant solid to particles less than 1 millimeter in size.

The drying can be done by freeze drying, spray drying oven drying, convection or forced air drying. The calcium:citrate:malate ratio can vary.

DETAILED DESCRIPTION OF THE INVENTION

As used herein the term, "calcium citrate malate" refers to a mixture of calcium, citrate and malate. The specific ratios will be defined as the ratio of calcium to citrate to malate. All ratios of calcium, citrate and malate are on a mole basis. The calcium citrate malate is referred to herein as CCM.

As used herein, the term "malic acid" refers to the mixture of the D and the L isomers, i.e. malic acid is optically active and the racemic mixture is used herein. D-malic acid and L-malic acid can be used.

As used herein the term "metastable" means that the material is not at equilibrium, and is a mixture of various crystalline and non-crystalline forms and solid solutions of the calcium ions, citrate ions and malate ions as well as salts of these materials. While the reaction kinetics would indicate that the calcium neutralizes all of the acid groups of citric and malic acid, or partially neutralizes depending on the ratio of the calcium to citric acid to malic acid, a molecular complex consisting of 2 moles of citrate and 3 moles of malate with 6 moles of calcium apparently does not form. But rather, an equilibrium is established between the salts and, when prepared by the process herein, a complex salt is formed. This complex salt is distinct from pure calcium citrate or pure calcium malate or simple mixtures thereof. The salt may be crystalline or micro crystalline, but may also represent an amorphous form or may contain solid solutions of calcium, citrate and malate ions.

This complex is thermodynamically an intermediate formed in the preparation of a dry form. Occasionally this reaction produces a product which is the most thermodynamically stable and is practically insoluble. The exact reason for this is not known.

The metastable materials probably have more than 1 crystalline state reflected by the presence of multiple hydration states. In addition, there are likely to be significantly different arrangements of the citrate and malate within the material. The physical and chemical data of these salts are consistent with the theory that there are non-crystalline regions within the powdered material which can hydrate to the point of behaving like a solution. It is important for the solubility characteristics of the calcium citrate malate that the apparent metastable structure be achieved.

Therefore, while the X-ray diffraction pattern indicates that there is some crystallinity, and that this material is different from calcium citrate or calcium malate, the exact structure of the material is not known.

The calcium citrate malate herein can exist in several states of hydration with from 2 to as many as 16 to 20 water molecules (e.g. a 6:2:3:8$H_2$O moiety exists).

A. Process for Making Metastable Calcium Citrate Malate

The calcium citrate malate is prepared by adding a calcium salt, as a powder or as an aqueous solution, to an aqueous solution of citric acid and malic acid in the mole ratio necessary to make the desired calcium citrate malate material. When calcium carbonate is used, carbon dioxide evolves as the calcium carbonate is neutralized by the citric and malic acids. The reaction is stirred until the carbon dioxide stops evolving and it appears that the materials have reacted. It should be recognized that not all of the carbon dioxide will evolve; some will dissolve in the water. When calcium oxide is used, or another source of calcium is used, the material is stirred until the acids have been neutralized.

The malic acid can be in solution and the citric acid and calcium source added to it at the same time or separately. It is preferable to add the calcium source to the solution of citric and malic acids. In other words adding malic acid to a premix consisting of calcium and citrate ion mixture, is not acceptable since calcium citrate forms readily and precipitates. This prevents the formation of the metastable CCM salt.

Preferably, calcium carbonate is used as the calcium source. Other sources include calcium oxide and calcium hydroxide. Calcium chloride, calcium phosphate and calcium sulphate are not suitable for use herein since the anions make an acid solution, i.e. hydrochloric acid, sulfuric and phosphoric acid, respectively. They also affect the flavor of the calcium citrate malate.

A solid forms during the mixing of the calcium oxide or calcium hydroxide with the citric and malic acid. When these materials are used, it is necessary to mix the solution until all of the calcium appears to have dissolved. The calcium citrate malate complex will precipitate when its solubility is exceeded.

The preferred method of preparation is to prepare a highly concentrated aqueous solution of the calcium citrate malate which quickly and efficiently forces metastable calcium citrate malate out of solution. Concentrations of from 20% to 75% (weight of reactants) in water are preferred. More preferably the concentration is from 40% to 65%.

The reaction temperature is from ambient (20°C) to 80°C. Preferably the temperature of the reaction is in the range of 30°C to 80°C. Most preferably it is from 40°C to 60°C.

This total mixture, which contains a supernatant CCM complex in solution as well as a solid can be dried. The solid can also be removed from the supernatant solution by filtration, centrifugation, or decantation and then the solid dried.

Drying can be done by forced air drying, convection drying, oven drying, freeze drying or spray drying. No matter what form of drying is used, the temperature of the drying should be less than 100°C. Above 100°C, the calcium citrate malate decomposes. When it decomposes, a less soluble mixture is made and the ratio of calcium to citric and malic acid is changed.

Preferably, when using forced air drying, the drying is accomplished on a thin layer of product at between 60°C and 85°C. The product is layered to between (0.05 inches) 0,127 cm and (0.5 inches) 1,27 cm thick.

In spray drying, the solution and solid mixture is sprayed into a hot air column at 60°C to 85°C. The pressure in the column is 79 993 to 119 990 Pa (600 to 900 millimeters of mercury.)

Vibratory freeze drying, or other conventional freeze drying techniques can also be used. The mixture is placed on a tray at a thickness of from about .01 to about 1 inch and frozen. A vacuum of 1,33 to 133,3 Pa (.01 to 1 millimeters of mercury) is used and a temperature of less than 25°C is maintained during the freeze drying operation.

The material is preferably dried until the amount of free or unbound water is less than 5%. Free water is water that is not a part of a CCM hydrate. The calcium citrate malate can form stable hydrate salts. The level of water, either as free water or as the hydrate is important for microbial stability and for handling. Too much water inhibits the ability to grind or tablet the material.

When forced air drying or freeze drying is used, the dried material is ground using any conventional grinding equipment and then sieved to a particle size of less than 1 millimeter. This particle size makes it easier to dissolve in foods and beverages and also represents a good particle size for tableting. Grinding and sieving should be done under anhydrous conditions or at low humidities to keep the metastable calcium citrate malate from rehydrating.

The ratio of calcium to citric acid to malic acid in the final product will depend upon the reactants used. Generally, the preparation of this material starting with pure calcium carbonate, citric acid and malic acid and drying the entire solution, including the supernatant liquid will produce a calcium citrate malate salt of the same ratio of the materials mixed. In making calcium citrate malate salts wherein the mole ratio of calcium are above 5, e.g. 6:2:3, 8:2:5 and 7:2:4, it is important to include the supernatent in the material to be dried since lower ratio salts could drop out of solution. The exact formula for the salts can be derived by measuring the percent of calcium, and the percentage of citric and malic acid in the solid material. It is important to remember that water also has to be analyzed since these materials readily form hydrate salts. The calcium level can be determined by atomic absorption measurements.

B. Description of the Product

The calcium citrate malate/metastable salt represents a soluble form of calcium, which is considerably more soluble than calcium citrate, calcium malate, or calcium carbonate. In addition, the calcium citrate malate prepared herein is considerably more soluble in dilute acid solutions.

The process herein can be used to make a variety of calcium citrate malate compositions. The mole ratio of calcium to citrate and malate determines the composition of the salts. The preferred salts for solubility and therefore for bioavailability are the neutral and acidic salts.

Neutral metastable calcium citrate malate compositions having the formula:

$3x + 2y = 2z$ wherein $x$ is the moles of citrate, $y$ is the moles of malate and $z$ is the moles of calcium. When $z$ is greater than 5, these metastable compositions exhibit enhanced solubility and functionality. The 6:2:3 salt is more soluble than 5:2:2 and other neutral salts have shown even better solubility. The ratio derived from this expression is expressed as integers and the ratio is reduced to the lowest common denominator. For example, 12:4:6 = 6:2:3; 6:2:3 is not expressed as 3:1:1.5.

The enhanced solubility of these salts is unexpected since one would not expect the anions and the ratio of calcium to anions to play such a key role in solubility. Table I lists the preferred neutral salts and their solubility expressed as calcium in solution per weight of calcium citrate malate used. The exact method for determining this is described hereinafter.

4

Table I

| Calcium Citrate Malate | Class | % Ca w/w CCM Solubility |
|---|---|---|
| 6:2:3 | Neutral | 91 |
| 5:1:1 | Basic | 45 |
| 5:2:2 | Neutral | 73 |
| 8:2:5 | Neutral | 89 |
| 4:2:3 | Acid | 100 |

The acidic salts, those wherein the levels of citric and malic acids is greater than the level of calcium $3x+2y>2z$ are also more soluble and therefore preferred compositions in terms of solubility and bioavailability.

The basic materials, i.e. those in which calcium is in excess $(3x+2y<2z)$, are not preferred. These tend to be less soluble (see Table I) and also contain excess amounts of calcium starting material. The method of solubility used to determine the data in Table I is described in the Analytical Methods section below. Using this method, calcium carbonate has a solubility of 0 and calcium citrate is 13, whereas calcium malate is 66.

A 6:2:3 calcium citrate malate solid prepared by the process herein is more soluble in either 1% or 10% acetic acid at a concentration of 500 milligrams of calcium per 50 milliliters (ml) of water than calcium citrate or calcium malate.

The following table lists the solubility of calcium salts in water (25°C):

| Calcium Salt | g Salt/100 ml $H_2O$ | Reference |
|---|---|---|
| CCM 6:2:3 | 1.10 | |
| Calcium Malate • 3 $H_2O$ | 0.4, (0.31) | 2, (1) |
| Calcium Citrate | 0.096 | 1 |
| Calcium Oxide | 0.1 | 1 |
| Calcium Hydroxide | 0.1 | 1 |
| Calcium Carbonate | 0.0056(.0014) | 1 (2) |
| CCM is metastable calcium citrate of this invention. | | |

(1) "Solubilities", 4th Edition, by W. F. Linke
(2) CRC Handbook of Chemistry and Physics, 67th Edition, (American Chemical Society)

All ratios are on a mole basis, and all percentages are by weight basis unless otherwise specified. The following examples illustrate the preparation of the materials herein, and are exemplary and are not meant to limit the invention. The 6:2:3 is prepared as in Example I.

EXAMPLE I

| Materials | Amount (g) |
|---|---|
| Calcium Carbonate (99+% purity) | 300 |
| Citric Acid (Anhydrous, powder) | 192 |
| Malic Acid (DL, practical) | 201 |
| Distilled/Deionized Water | 1000 |

Procedure

To prepare about 500 grams of CCM (6:2:3) powder:

Dissolve 192 g of citric acid and 201 g of malic acid in 1000 ml of distilled-deionized water in a 2 liter glass beaker and stir with a teflon coated magnetic stir bar until solution is clear (about 5 minutes). Carefully

EP 0 533 724 B1

add 300 g of $CaCO_3$ to the acids solution at ambient temperature. This solid is added quickly, but at a rate slow enough to control the carbon dioxide evolution and to avoid overflowing the beaker. The mixture is stirred for 3 hours at room temperature. After 3 hours, the mixture is transferred in total to a 12 inch by 16 inch stainless tray to yield a solution fill level of approximately 0.5-0.75 inch. The mixture is dried in an 80°C forced air oven (model Blue-M Stabil-Therm) for 19 hours.

The tray is removed and allowed to cool to room temperature. The CCM solid is ground to pass through a #20 screen (84 microns particle size) with a Wiley mill.

The calcium citrate malate solid is stored in a cool, dry place to avoid increasing the level of hydration. The calcium level of the product is 20.73% corresponding to the octahydrate form.

EXAMPLE II

A metastable 6:2:3 calcium citrate malate is prepared as in Example I except that the material is freeze dried at a temperature of 25°C and a vacuum 0.01 millimeters of mercury.

C. Analytical Methods

1. Solubility
Determine % Ca wt/wt in CCM (Calcium Citrate Malate) sample by analysis.
Calculate g CCM to achieve 0.1 g Ca in the sample.
Add 50 ml water at room temperature (about 20°C) to the sample and stir 30 minutes.
The sample slurry is then filtered rapidly through Whatman #4 filter paper (3") lined Buchner funnel until all visible water is through, a slight vacuum is used. The filtrate is diluted to 100 ml with 5% HCl solution in water.
A 200 ul sample of this solution is diluted to 50 ml with 1 ml 5% lanthanum (as lanthanum oxide) and 5% HCl. The Ca is determined by atomic absorption. This is the soluble calcium.
The residue (on filter paper) is dissolved in 5% HCl and diluted to 200 ml. The calcium level is determined by atomic absorption as above. This is the insoluble Ca.
The total of soluble and insoluble Ca should equal 100 mg. The percent soluble calcium is easily calculated.

2. Ca Content of CCM
Dissolve 100 mg CCM in 5% HCl and dilute to 100 ml with 5% HCl. To 1 ml of this solution is added 1 ml 5% lanthanum (as lanthanum oxide) and the sample diluted to 100 ml with 5% HCl.
The Ca level is measured by atomic absorption using the following procedure:

Equipment - Perkin-Elmer Atomic Absorption Spectrophotometer Model 3030.

Stock Standard Solution
Calcium, 500 mg/L. To 1.249 g of primary standard calcium carbonate, $CaCO_3$, add 50 ml of deionized water. Add dropwise a minimum volume of 5% HCl (approximately 10 ml), to effect complete solution of the $CaCO_3$. Dilute to 1 liter with deionized water.

Light Sources
When using the calcium 239.9 nm line, the use of a multi-element (Ca-Mg) or (Ca-Mg-Al) hollow cathode lamp with a quartz window is recommended.

Flame Adjustment
The absorption of calcium is dependent on the fuel/air ratio and the height of the light beam above the burner. Although maximum sensitivity is obtained with a reducing (fuel-rich) flame, an oxidizing (fuel-lean) flame is recommended for optimum precision.

Other Flames
Calcium determination appears to be free from chemical interferences in the nitrous oxide-acetylene flame. Ionization interferences should be controlled by the addition of alkali salt (0.1% or more potassium as chloride) to samples and standards. It is probably preferable to determine calcium in a nitrous oxide-acetylene flame, especially in samples containing large amounts of silica.

Interferences
Slight ionization occurs in the air-acetylene flame, and can be controlled by the addition of an alkali salt (0.1% or more potassium as chloride) to samples and standards. Calcium sensitivity is reduced in the presence of elements which give rise to stable oxysalts. These elements include aluminum, beryllium, phosphorus, silicon, titanium, vanadium, and zirconium. This effect is reduced by the addition of 0.1 -1.0% lanthanum or strontium.

Standard Atomic Absorption Conditions for Ca

6

| Wavelength (nm) | Slit (nm) | Sensitivity (mg/L) | Linear Range (mg/L) |
|---|---|---|---|
| 422.7 | 0.7 | 0.092 | 5.0 |
| 239.9 | 0.7 | 13.0 | 800.0 |

Recommended Flame: Air-acetylene, oxidizing (lean, blue).
Sensitivity with a flow spoiler and N20-C2H2 flame at 422.7 nm: 0.048 mg/L.
Standard Flame Emission Conditions for Ca

| Wavelength (nm) | Slit (nm) | Flame |
|---|---|---|
| 422.7 | 0.2 | Nitrous oxide-acetylene |

The calcium is determined by comparing the absorption with standard calcium solutions.

3. Determination of Citric and Malic Acids in Calcium Citrate Malate (CCM)

A CCM sample is dissolved in dilute HCl and the dissolved organic acids are determined by ion exclusion HPLC (high pressure liquid chromatography).

Reagents (99 + or certified grades)
Citric Acid, anhydrous
d,l Malic Acid
Tricalcium Dicitrate Tetrahydrate
Calcium Malate Trihydrate
0.2N HCl
1N $H_2SO_4$
Water
HPLC

| Pump | Kratos Spectroflow 400 |
|---|---|
| Injector | Waters WISP 710B |
| In-Line Filter | Rainin 0.5 u |
| Column and Precolumn | Bio Rad HPX-87H, 300x7.8 mm |
| Detector | Kratos Spectroflow 783 |
| Integrator | Hewlett Packard 3390A |

Procedure
A. Preparation of Stock Solutions
HPLC Mobile Phase - Pipet 1.5 mL of 1N $H_2SO_4$ into a 1L volumetric flask. Dilute to volume with HPLC grade water and shake to mix. Filter through an 0.45 u filter and degas under vacuum.
B. Preparation of Standard
Weigh accurately 0.025 g each of citric and malic acid into a 25 ml volumetric flask. Add mobile phase and shake to dissolve acids. Dilute to volume with mobile phase and shake to mix thoroughly.
C. Sample Preparation
1. Weigh accurately 0.05 g of CCM powder or put liquid sample into a 10 ml volumetric flask. Dissolve in 0.2N HCl. Dilute to 10 ml with 0.2N HCl and shake to mix thoroughly. If the sample does not completely dissolve, add additional HCl until dissolution is complete or repeat with a smaller sample.
2. Transfer a few milliliters to an autosampler vial for injection.
D. Chromatographic Procedure

7

| Mobile Phase | Isocratic, 0.0015N $H_2SO_4$ |
|---|---|
| Flow Rate | 0.8 mL/min |
| Injection Volume | 25 $\mu$L |
| Detector | UV 210 nm, 0.1 absorbance units full scale |

<u>Calculations</u>

Weight % Acid = (As/Astd) x Cs x 1000/SW

where As = peak area for acid in sample

Astd = peak area for acid in standard

Cs = concentration of acid in standard in mg/mL

for example if, for citric acid:

As = 3.26 EO6

Astd = 1.72 EO6

Cs = 1.02

SW = 51.4

then the weight % citric acid is

(3.26 EO6/1.72EO6) x 1.02 x 1000/51.4 a 37.6%

4. Hydrate Level

Since CCM samples decompose when heated above 100°C for extended periods, it is difficult to determine the water level by drying or dessication procedures. The water level of the sample is determined by subtracting the total calcium, citric and malic acid concentrations from 100. Any remaining material is water.

Most dissolved carbon dioxide from the preparation is lost during the drying so it is negligible. Any carbonate anions remaining in "basic CCMs" should be accounted for before calculating the water level.

## Claims

1. A process for forming a metastable calcium citrate malate having a solubility in water at about 20°C of 75% or more weight percent calcium per calcium citrate malate on a 100 mg basis comprising:

   (a) adding a calcium source selected from the group consisting of calcium hydroxide, calcium carbonate, or calcium oxide to citric and malic acid in water, preferably wherein the mole ratios of calcium is from 2 to 10, of citric is from 1 to 3 and of malic acid is from 1 to 5;

   (b) mixing this solution at 20°C to 80°C until the citric and malic acids are neutralized by the calcium, preferably at a temperature of from 30°C to 80°C;

   (c) drying the mixture at a temperature of less than 100°C; and

   (d) grinding the resultant solid to particles less than 1 millimeter in size.

2. A process according to claim 1 or 2 wherein the mole ratio of calcium is from 4 to 8 and the calcium citrate malate is neutral or acidic.

3. A process according to claim 3 wherein the drying is freeze drying at a temperature of less than 25°C at a pressure of from 1,33 to 133,3 Pa (0.01 to 1 mm Hg.)

4. A process according to claim 3 wherein the mixture is spray dried at a temperature of less than 90°C and a pressure of from 79 993 to 119 990 Pa (600 millimeter mercury to 900 millimeters of mercury.)

5. A process according to claim 1, 2, 4 or 5 wherein the concentration of the calcium, citric and malic acids are from 20% to 75% in the water of step (a), and preferably wherein the citric acid is added simultaneously with the calcium source to malic acid in water.

6. A metastable calcium citrate malate complex of the formula $3x + 2y = 2z$ wherein 3x,2y and 2z are integers and wherein x is the moles of citrate, y is the moles of malate and z is the moles of calcium and wherein 2 z is greater than 5; said complex having a solubility of 75% or more weight percent calcium per calcium citrate malate on a 100 mg basis, measured at room temperature (about 20°C).

**Patentansprüche**

1. Verfahren zum Ausbilden eines metastabilen Calciumcitratmalates mit einer Löslichkeit in Wasser bei etwa 20°C von 75 Gew.-% oder mehr an Calcium pro Calciumcitratmalat, bezogen auf 100 mg hievon, welches Verfahren die folgenden Stufen umfaßt:

   (a) Zugabe einer Calciumquelle, welche aus einer Gruppe ausgewählt ist, die aus Calciumhydroxid, Calciumcarbonat oder Calciumoxid besteht, zu Zitronensäure und Apfelsäure in Wasser, wobei in den Molverhältnissen zwischen dem Calcium, der Zitronensäure und der Apfelsäure die Molanteile für das Calcium 2 bis 10 betragen, die Molanteile für die Zitronensäure 1 bis 3 betragen und die Molanteile für die Apfelsäure 1 bis 5 betragen;

   (b) Vermischen dieser Lösung bei 20°C bis 80°C so lange, bis die Zitronensäure und die Apfelsäure durch das Calcium neutralisiert worden sind, vorzugsweise bei einer Temperatur von 30°C bis 80°C;

   (c) Trocknen des Gemisches bei einer Temperatur von unter 100°C; und

   (d) Zerkleinern des so entstandenen Feststoffes auf Teilchen mit einer Größe von unter 1 mm.

2. Verfahren nach Anspruch 1, wobei in dem obgenannten Molverhältnis die Molanteile für das Calcium 4 bis 8 betragen und das Calciumcitratmalat neutral oder sauer ist.

3. Verfahren nach Anspruch 2, wobei das Trocknen ein Gefriertrocknen bei einer Temperatur von unter 25°C und bei einem Druck von 1,33 bis 133,3 Pa (0,01 bis 1 mm Hg) ist.

4. Verfahren nach Anspruch 2, wobei das Gemisch bei einer Temperatur von unter 90°C und bei einem Druck von 79 993 bis 119 990 Pa (600 mm Hg bis 900 mm Hg) sprühgetrocknet wird.

5. Verfahren nach einem der Ansprüche 1, 2, 3 oder 4, wobei die Konzentration des Calciums, der Zitronensäure und der Apfelsäure in dem Wasser der Stufe (a) 20 % bis 75 % beträgt und wobei die Zitronensäure vorzugsweise gleichzeitig mit der Calciumquelle zu der Apfelsäure in Wasser zugegeben wird.

6. Metastabiler Calciumcitratmalatkomplex der Formel $3x + 2y = 2z$, worin $3x$, $2y$ und $2z$ ganze Zahlen sind, und worin $x$ die Molanzahl des Citrates bedeutet, $y$ die Molanzahl des Malates bedeutet, und $z$ die Molanzahl des Calciums bedeutet und worin $2z$ größer als 5 ist; und wobei dieser Komplex eine bei Raumtemperatur (etwa 20°C) gemessene Löslichkeit von 75 Gew.-% oder mehr an Calcium pro Calciumcitratmalat, bezogen auf 100 mg hievon, aufweist.

**Revendications**

1. Procédé pour former un citrate-malate de calcium métastable ayant une solubilité dans l'eau à environ 20°C de 75%, ou plus, pourcentage en poids de calcium rapporté à 100 mg de citrate-malate de calcium, consistant:

   (a) à ajouter une source de calcium choisie dans le groupe formé par l'hydroxyde de calcium, le carbonate de calcium ou l'oxyde de calcium à de l'acide citrique et malique dans l'eau, de préférence dans laquelle le rapport molaire du calcium est de 2 à 10, celui de l'acide citrique est de 1 a 3 et celui de l'acide malique est de 1 à 5;

   (b) à mélanger cette solution à une température de 20°C à 80°C jusqu'à ce que les acides citrique et malique soient neutralisés par le calcium, de préférence à une température de 30°C à 80°C;

   (c) à sécher le mélange a une température inférieure à 100°C; et

   (d) à écraser le solide résultant en particules de taille inférieure à 1 millimètre.

2. Procédé selon la revendication 1 ou 2, dans lequel le rapport molaire du calcium est de 4 à 8 et le citrate-malate de calcium est neutre ou acide.

3. Procédé selon la revendication 3, dans lequel le séchage est une lyophilisation à une température inférieure a 25°C sous une pression de 1,33 à 133,3 Pa (0,01 à 1 mm (Hg).

4. Procédé selon la revendication 3, dans lequel le mélange est séché par atomisation à une température inférieure à 90°C et sous une pression de 79 893 à 119 990 Pa (600 millimètres de mercure à 900 millimètres de mercure).

5. Procédé selon la revendication 1, 2, 4 ou 5, dans laquelle la concentration en calcium, en acides malique et citrique est de 20% à 75% dans l'eau de l'étape (a), et de préférence dans lequel l'acide citrique est ajouté simultanément avec la source de calcium à l'acide malique dans l'eau.

6. Complexe de citrate-malate de calcium métastable de formule $3x + 2y = 2z$, dans lequel $3x$, $2y$ et $2z$ sont des nombres entiers et dans lequel $x$ représente les moles de citrate, $y$ représente les moles de malate et $z$ représente les moles de calcium et dans lequel $2z$ est supérieur à 5; ledit complexe ayant une solubilité de 75%, ou plus, pourcentage en poids de calcium rapporté à 100 mg de citratemalate de calcium, mesurée à la température ambiante (environ 20°C).